# EUROPÄISCHE PATENTANMELDUNG

(11) **EP 1 886 651 A1**
(43) Veröffentlichungstag der Anmeldung: **13.02.2008**
(21) Anmeldenummer: 07013431.7
(22) Anmeldetag: 10.07.2007
(51) Int. Cl.: A61F 2/90

(54) **Stent mit einer Struktur aus einem biokorrodierbaren metallischen Werkstoff**

(30) Priorität: 07.08.2006 DE 102006038242
(71) Anmelder: BIOTRONIK VI Patent AG, 6341 Baar (CH)
(72) Erfinder: Müller, Heinz, Dr., 91052 Erlangen (DE); Rzany, Alexander, Dr., 90449 Nürnberg (DE)
(74) Vertreter: Lindner-Vogt, Karin L.

(57) **Zusammenfassung**

Die Erfindung betrifft einen Stent mit einer Struktur aus einem biokorrodierbaren metallischen Werkstoff, die aus einer Vielzahl miteinander in Verbindung stehender Stegabschnitte besteht, wobei
(i) die Struktur eine Stützstruktur aus einer Anzahl miteinander in Verbindung stehender erster Stegabschnitte aufweist, die ausgelegt sind eine nach einer Expansion des Stents die Gefäßwand abstützende oder die mechanische Integrität des Stents bewahrende Funktion für einen vorgebbaren Zeitraum wahrzunehmen; und
(ii) wenigstens ein zweiter Stegabschnitt vorhanden ist,
(a) der direkt mit einem ausgewählten ersten Stegabschnitt der Stützstruktur elektrisch leitend verbunden ist,
(b) der keine nach der Expansion des Stents die Gefäßwand abstützende oder die mechanische Integrität des Stents bewahrende Funktion für den vorgegebenen Zeitraum wahrnimmt und
(c) dessen Elektrodenpotential E₂ durch eine mechanische Beanspruchung des zweiten Stegabschnittes während oder vor der Expansion des Stents derart herabgesetzt ist, dass es geringer ist als ein Elektrodenpotential E₁ des ausgewählten ersten Stegabschnitts nach der Expansion des Stents und als Opferanode dient.

## Beschreibung

Die Erfindung betrifft einen Stent mit einer Struktur aus einem biokorrodierbaren metallischen Werkstoff.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen einen rohrförmigen Grundkörper durch den der Blutfluss ungehindert weiterläuft und dessen Umfangswandung eine Stützfunktion für die Gefäßwand wahrnimmt. Der Grundkörper ist häufig als gitterartige Struktur verwirklicht, mit einer Vielzahl von einzeln und miteinander verbundenen Stegabschnitten. Weiterhin muss die Designvorgabe für die gitterartige Struktur es erlauben, den Stent in einem komprimierten Zustand mit - kleinem Außendurchmesser bis zur zu behandelnden Engstelle des jeweiligen Gefäßes einzuführen und dort beispielsweise mit Hilfe eines Ballonkatheters so weit aufzuweiten, dass das Gefäß den gewünschten, vergrößerten Innendurchmesser aufweist. Um unnötige Gefäßbeschädigungen zu vermeiden, sollte der Stent nach dem Aufweiten und Entfernen des Ballons nicht oder allenfalls in geringem Umfang elastisch zurückfedern, so dass der Stent beim Aufweiten nur wenig über den gewünschten Enddurchmesser hinaus geweitet werden muss. Weitere konstruktive Anforderungen sind eine gleichmäßige Flächenabdeckung und eine Struktur, die eine gewisse Flexibilität in Bezug auf die Längsachse des Stents erlaubt. Konstruktiv lassen sich die einzelnen, eine gitterartige Struktur bildenden Stegabschnitte gliedern in solche mit Stützfunktion für die Gefäßwand und tragender Funktion (d. h. die mechanische Integrität des Implantats gewährender Funktion) und solche ohne Beteiligung an den genannten konstruktiven Aufgaben. Das Geflecht der ersteren Stegabschnitte wird nachfolgend als Tragstruktur bezeichnet. In der Praxis wird der Stent zur Wahrnehmung der genannten konstruktiven Vorgaben zumeist aus einem metallischen Werkstoff geformt.

Neben den mechanischen Eigenschaften eines Stents sollte dieser aus einem biokompatiblen Werkstoff bestehen, um Abstoßungsreaktionen zu minimieren. Derzeit werden bei etwa 70 % aller perkutanen Interventionen Stents eingesetzt, in 25% aller Fälle kommt es jedoch zu einer in-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatzpunkt ist die Verwendung biokorrodierbarer Metalllegierungen, denn zumeist ist eine dauerhafte Stützfunktion durch den Stent nicht notwendig; das zunächst geschädigte Gefäß kann sich regenerieren. Dementsprechend wird beispielsweise in DE 197 31 021 A1 vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1 % bekannt, die sich insbesondere zur Herstellung einer Endoprothese, z. B. in Form eines selbstexpandierenden oder ballonexpandierbaren Stents, eignet.

Biokorrodierbare Implantate stellen somit einen aussichtsreichen Ansatz zur Minderung der Restenoserate dar. Ein Problem bei der Realisation derartiger Systeme ist das Korrosionsverhalten des Implantats. So sollte eine Fragmentbildung durch den Korrosionsprozess nach Möglichkeit bis zum Einwachsen des Implantats in die Gefäßwand unterbunden sein. Weiterhin sollte die Stützfunktion über den Zeitraum der therapeutischen Vorgabe aufrecht erhalten werden. Die oben genannten konstruktiven Vorgaben lassen eine freie Anpassung des Stentdesigns in Bezug auf sein Korrosionsverhalten nicht zu - es müssen Kompromisse eingegangen werden.

Es hat sich nun gezeigt, dass die einzelnen Stegabschnitte der Tragstruktur eines biokorrodierbaren Stents - auch wenn sie aus ein und demselben Material gleicher Materialstärke geformt sind - nicht gleichmäßig korrodieren. Die Ursache für dieses differenzierte Korrosionsverhalten mag in der unterschiedlichen mechanischen Beanspruchung der verschiedenen Stegabschnitte der Tragstruktur bei der Herstellung des Stents, dem Aufcrimpen des Stent auf einen Katheter sowie der Dilatation des Implantats am Ort der Läsion liegen. Ferner können die Materialdicken der einzelnen Stegabschnitte der Tragstruktur variieren und das Korrosionsverhalten wird zudem maßgeblich von den am Implantationsort herrschenden lokalen Bedingungen bestimmt; zum Beispiel werden die Stegabschnitte lumenseitig schneller abgebaut, da der Blutfluss die für Teilprozesse der Korrosion bedeutsame Konzentration von Magnesiumhydroxid und Wasserstoff in der Phasengrenzfläche Elektrolyt/Implantat herabsetzt. Insgesamt hat dies zur Folge, dass in einigen Bereichen der Tragstruktur die Korrosion beschleunigt ist, was wiederum dazu führen kann, dass die Tragstruktur nicht über die gewünschte Dauer aufrechterhalten werden kann.

Der vorliegenden Erfindung liegt die Aufgabe zugrunde, die geschilderten Nachteile des Standes der Technik zu überwinden. Insbesondere soll ein Stent mit einem verbesserten Korrosionsverhalten seiner Tragstruktur bereitgestellt werden.

Diese Aufgabe wird durch den erfindungsgemäßen Stent mit einer Struktur aus einem biokorrodierbaren metallischen Werkstoff gelöst. Die Struktur besteht dabei aus einer Vielzahl miteinander in Verbindung stehender Stegabschnitte, wobei
(i) die Struktur eine Tragstruktur aus einer Anzahl miteinander in Verbindung stehender erster Stegabschnitte aufweist, die ausgelegt sind eine nach einer Expansion des Stents die Gefäßwand abstützende oder die mechanische Integrität des Stents bewahrende Funktion für einen vorgebbaren Zeitraum wahrzunehmen; und
(ii) wenigstens ein zweiter Stegabschnitt vorhanden ist,
   (a) der direkt mit einem ausgewählten ersten Stegabschnitt der Stützstruktur elektrisch leitend verbunden ist,
   (b) der keine nach der Expansion des Stents die Gefäßwand abstützende oder die mechanische Integrität des Stents bewahrende Funktion für den vorgegebenen Zeitraum wahrnimmt und
   (c) dessen Elektrodenpotential E₂ durch eine mechanische Beanspruchung des zweiten Stegabschnittes während oder vor der Expansion des Stents derart herabgesetzt ist, dass es geringer ist als ein Elektrodenpotential E₁ des ausgewählten ersten Stegabschnitts nach der Expansion des Stents.

Der Erfindung liegt die Erkenntnis zugrunde, dass eine unterschiedliche mechanische Beanspruchung ein und desselben Werkstoffs zu einer Änderung des Elektrodenpotentials in den jeweils unterschiedlich belasteten Bereichen des Werkstoffs führt. Erfindungsgemäß ist nun vorgesehen, diese Potentialdifferenz zu nutzen, um die Tragstruktur des Stents in einer ersten Phase der Degradation zu stabilisieren. Der genannte Zeitraum beginnt unmittelbar nach der Implantation des Stents und endet zu einem vorgebbaren Zeitpunkt, der den therapeutischen Vorgaben und Anforderungen an die Sicherheit entspricht. Vorzugsweise erstreckt sich dieser Zeitraum über 2 bis 6 Wochen unmittelbar nach der Implantation. In der Regel ist der Stent innerhalb dieses Zeitraums in die Gefäßwand eingewachsen und diese ist soweit regeneriert, dass eine weitere Stützfunktion nicht mehr notwendig ist.

Eine Tragstruktur im Sinne der Erfindung fasst die Stegabschnitte zusammen, die eine Stützfunktion für die Gefäßwand über der vorgegebenen Zeitraum und die Konstruktion tragende Funktion (d. h. die mechanische Integrität des Implantats bewahrende Funktion) über den vorgegebenen Zeitraum wahrnehmen. Dies sind insbesondere die Stegabschnitte, ohne die die Stützfunktion und tragende Funktion nicht mehr den Erfordernissen am Implantationsort genügt.

Durch eine gezielte mechanische Belastung werden nun im Elektrodenpotenzial herabgesetzte Stegabschnitte in bestimmten Bereichen der Tragstruktur bereitgestellt. Diese besonderen Stegabschnitte sind so geschaffen, dass ihr Elektrodenpotential - nach der Dilatation des Stents - niedriger ist als das Elektrodenpotenzial des jeweiligen Stegabschnittes der Tragstruktur, mit dem sie (elektrisch leitend) verbunden sind. Der Stegabschnitt mit niedrigerem Elektrodenpotenzial agiert somit als Opferanode, das heißt, der mit ihm verbundene Stegabschnitt der Tragstruktur wird temporär stabilisiert, bis die Opferanode vollständig oder größtenteils abgebaut ist. Über die Masse der als Opferanode agierenden Stegabschnitte kann eine Dauer des stabilisierenden Effektes beeinflusst werden und zwar mit der Maßgabe, dass mindestens der oben genannte vorgegebene Zeitraum sichergestellt ist. Natürlich hat der als Opferanode agierende Stegabschnitt nicht selbst eine tragende Funktion wahrzunehmen oder zur Aufrechterhaltung der mechanischen Integrität des Implantats zu dienen. Ein besonderer Vorteil des erfindungsgemäßen Konzepts liegt darin, dass für das gesamte Implantat der gleiche biodegradierbare metallische Werkstoff verwendet wird und eine Feinabstimmung der Werkstoffeigenschaften hinsichtlich des Korrosionsverhaltens durch eine unterschiedlich starke mechanische Belastung des Materials in bestimmten Stegabschnitten erreicht wird.

Über die Ursachen für die durch mechanische Beanspruchung resultierende Minderung des Elektrodenpotentials kann bisher nur spekuliert werden: es mögen Effekte, wie die Veränderungen im Gefüge des Metalls/der Legierung bei mechanischer Last, die daraus resultierende Veränderung an der Grenzfläche Metall/Elektrolyt, die Veränderung thermodynamischer Potentiale für Elektronendurchtrittsprozesse über die Phasengrenze Metall/Elektrolyt, der lokale Transport von Elektronen in dem Metall, die Beteiligung von entstehendem Wasserstoff eine Rolle spielen. Das grundlegende Prinzip entspricht dem eines kathodischen Korrosionsschutzes von Metallen, wie er zum Beispiel bei einem Überzug von Eisen mit unedlen Metallen verwendet wird (Verzinken). Die unedlen Bereiche entstehen bei der vorgeschlagenen Idee jedoch nicht durch Beschichtung mit einem anderen Metall oder durch Anbringen einer Opferanode aus einem anderen Metall sondern durch unterschiedlich große mechanische Spannungen in unterschiedlichen Bereichen ein und desselben Metalls/Legierung. Durch den kathodischen Schutz wird vermutlich erreicht, dass in den Bereichen mit geringeren internen mechanischen Spannungen nur noch bzw. deutlich vermehrt kathodische Reaktionen wie eine Wasserstoffentwicklung bzw. eine Sauerstoffreduktion möglich sind, während die anodische Metallauflösung in Zonen mit hohen internen mechanischen Spannungen konzentriert auftritt.

Unter Elektrodenpotential - das nur als Spannung (Elektrodenspannung) gegen eine Bezugselektrode messbar ist - versteht man das elektrische Potential eines Metalls oder eines elektronenleitenden Festkörpers in einem Elektrolyten. Stehen zwei Elektroden im Kontakt mit einem Elektrolyten, lässt sich zwischen ihnen eine elektrische Spannung messen. Das Elektrodenpotenzial (Symbol: E) gibt an, welche elektrische Spannung eine Elektrode liefern kann oder welche Spannung benötigt wird, um - beispielsweise bei einer Elektrolyse - einen bestimmten Zustand aufrecht zu erhalten. Die Spannung zwischen zwei Polen ist definiert als die elektrostatische Energie, die man benötigt, um eine Coulomb Ladung von einem Pol zum andern zu bewegen. Diese Energie kann man direkt messen, wenn man Ladungen im Vakuum, innerhalb eines Metalls oder zwischen zwei Metallpolen, bewegt. Wenn man aber eine Ladung, beispielsweise ein Elektron, aus einer Metallelektrode in eine Elektrolytlösung bringt, so ist die dafür benötigte Energie nicht nur durch elektrostatische, sondern auch durch chemische Wechselwirkungen des Elektrons mit dem Metall oder mit den Lösungsbestandteilen bestimmt. Das Elektrodenpotenzial E ist nun die Spannung der Elektrode, die gegenüber einer Bezugselektrode gemessen wird. Bezugselektroden sind Elektroden mit bekanntem Potenzial, das heißt mit bekanntem elektrochemischen Zustand. Der Wert des Elektrodenpotentials E wird in Volt (V) angegeben. Als Prüfmedium zur Bestimmung des Elektrodenpotentials dient für die vorliegenden Zwecke insbesondere künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l).

Es hat sich nun gezeigt, dass eine unterschiedliche mechanische Belastung des metallischen Werkstoffs zu Potentialdifferenzen von einigen Millivolt (mV) führt. Vorzugsweise liegt eine Differenz aus dem Elektrodenpotential E₁ des ersten Stegabschnittes und dem Elektrodenpotential E₂ des zweiten Stegabschnittes bei mehr als 5 mV. Insbesondere liegt die Potenzialdifferenz im Bereich von 5 mV - 100 mV. Bei den vorgegebenen Potenzialdifferenzen ist sichergestellt, dass ein wie oben beschriebener stabilisierender Effekt im Sinne einer Opferanode auftritt.

Das gegenüber dem zu stabilisierenden Stegabschnitt der Tragstruktur verminderte Elektrodenpotential E₂ des zweiten Stegabschnittes kann auf verschiedenen Wegen eingestellt werden: (i) im Fertigungsprozess des Stents erfolgt eine gezielte mechanische Belastung oder mechanisch belastete Stegabschnitte werden mit der Tragstruktur verbunden, (ii) beim Crimpen des Stents werden ausgewählte Stegabschnitte gezielt belastet, und (iii) die mechanische Belastung erfolgt im Zuge der Dilatation des Stents. Letztere Variante ist bevorzugt, da hierdurch das Fertigungs- und Crimpverfahren für den Stent vereinfacht ist.

Vorzugsweise ist der biokorrodierbare metallische Werkstoff eine biokorrodierbare Legierung, ausgewählt aus der Gruppe Magnesium, Eisen und Wolfram; insbesondere ist der biokorrodierbare metallische Werkstoff eine Magnesiumlegierung. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest <1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Die Legierungen der Elemente Magnesium, Eisen oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion des metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflusst die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

Die Erfindung wird nachfolgend anhand von Ausführungsbeispielen und den dazugehörigen Zeichnungen näher erläutert. Es zeigen:
- Figuren 1A und 1B: eine schematische Darstellung eines Ausschnitts aus einem Stent, der die erfindungsgemäßen Strukturen nach einer ersten Variante enthält; und
- Figur 2A und 2B: eine schematische Darstellung einer zweiten Variante der erfindungsgemäßen Struktur.

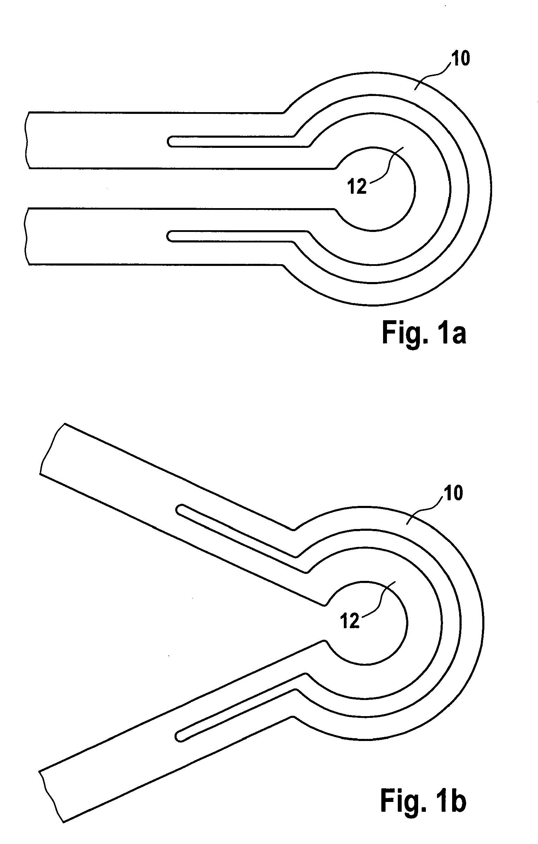

Figur 1A zeigt einen Ausschnitt aus der Struktur eines Stent im nicht expandierten Zustand und Figur 1B den selben Ausschnitt nach Expansion des Stents. Der Stent kann beispielsweise aus der biokorrodierbaren Magnesiumlegierung WE43 geformt sein (93 Gew.% Magnesium, 4 Gew.% Yttrium und 3 Gew.% Seltenerdmetalle außer Yttrium). Die Struktur eines solchen Stents besteht aus einer Vielzahl miteinander verbundener Stegabschnitte, die die Tragstruktur und andere konstruktive Elemente des Implantats ausbilden. Die Struktur kann beispielsweise miteinander über Stege verbundene Ringelemente oder helixförmig umlaufende Stege umfassen. In dieser Struktur lassen sich Stegabschnitte identifizieren, die ausgelegt sind, nach der Expansion des Stents eine die Gefäßwand abstützende oder die mechanische Integrität des Stents bewahrende Funktion für einen vorgebbaren Zeitraum wahrzunehmen. Es sei nun unterstellt, dass die Figuren 1A und 1 B einen Ausschnitt der Struktur zeigen, der Stegabschnitte dieser Tragstruktur enthält; hier einen ersten Stegabschnitt 10 der Tragstruktur als halbringförmig umlaufender Steg. Die Struktur enthält weiterhin einen zweiten Stegabschnitt 12 - in Form eines zweiten halbringförmigen Stegelementes - mit einem gegenüber dem ersten Stegabschnitt 10 geringeren Umfang.

Im nicht expandierten Zustand des Stents (Figur 1A) seien die Elektrodenpotenziale des ersten und zweiten Stegabschnittes als identisch unterstellt. Bei der Expansion des Stents werden die beiden Stegabschnitte 10, 12 jedoch unterschiedlich stark plastisch deformiert. Hieraus resultierte eine unterschiedlich starke Änderung des elektrochemischen Potentials. Der zweite Stegabschnitt 12 wird stärker plastisch deformiert und sein Elektrodenpotenzial E₂ sinkt in einem größeren Ausmaß als das Elektrodenpotential E₁ des ersten Stegabschnitts 10. Damit einhergehend ändert sich auch das Korrosionsverhalten der beiden Stegabschnitte 10, 12: Der zweite Stegabschnitt 12 wird in der Regel zügiger, da nun unedler, korrodieren. Dadurch, dass der erste und zweite Stegabschnitt 10, 12 miteinander elektrisch leitend verbunden sind, werden jedoch auch korrosive Prozesse stattfinden, die zu einer Beschleunigung der Korrosion des zweiten Stegabschnittes 12 und einer Hemmung/Verlangsamung der Korrosion am ersten Stegabschnitt 10 im Sinne eines Opferanodensystems führen. Die Potenzialdifferenz zwischen den beiden Stegabschnitten 10, 12 bewirkt also, dass der erste Stegabschnitt 10 temporär stabilisiert wird, und zwar bis der zweite Stegabschnitt 12 vollständig oder in größerem Umfang abgebaut ist. Somit kann der erste Stegabschnitt 10 seine Funktion in der Tragstruktur länger wahrnehmen.

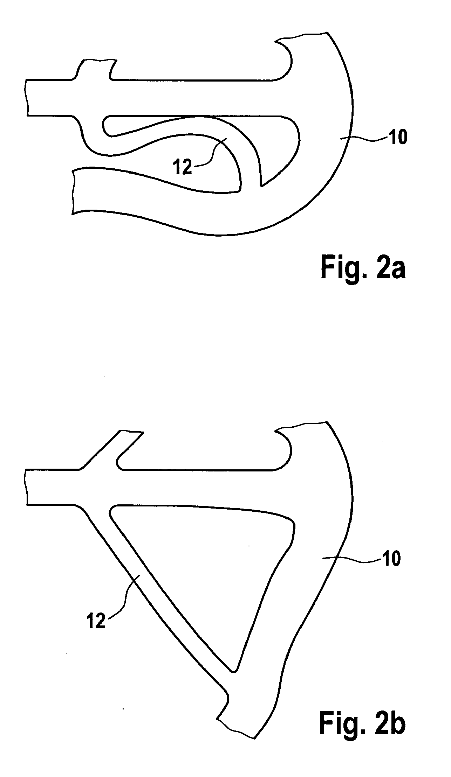

Den Figuren 2A und 2B ist eine weitere Variante der ersten und zweiten Stegabschnitte 10, 12 zu entnehmen. Die Funktionsweise entspricht dem ersten Ausführungsbeispiel, so dass auf die vorangehenden Ausführungen verwiesen wird. Der erste Stegabschnitt 10 wird hier als der Teil der Tragstruktur definiert, der zwischen den beiden Anbindungspunkten des zweiten Stegabschnitts 12 liegt. Der zweite Stegabschnitt 12 wird bei der Expansion des Stents am stärksten plastisch deformiert und kann entsprechend den vorherigen Ausführungen als Opferanode für den ersten Stegabschnitt 10 dienen, so dass dieser temporär in seiner Korrosionsrate gehemmt ist.

Bei diesen beiden Beispielen werden demnach konstruktionsbedingt unterschiedlich starke mechanische Spannungen in verschiedenen Bereichen einer Stentstruktur zum Zeitpunkt der Dilatation generiert, nämlich an den dargestellten ersten und zweiten Stegabschnitten 10, 12. Der zweite Stegabschnitt 12, der einer höheren mechanischen Belastung ausgesetzt wurde, hat nach der Dilatation ein niedrigeres Elektrodenpotential E₂ als der elektrisch leitend mit ihm verbundene erste Stegabschnitt 10 und agiert deshalb als Opferanode. Hierdurch ist die Degradation des ersten Stegabschnitts 10 temporär gehemmt.

Es ist auch denkbar, bereits zum Zeitpunkt der Herstellung durch Pressen des Stents in einer geeigneten Form mechanische Vorspannungen in Bereichen der Stentstruktur zu erzeugen (d. h. im zweiten Stegabschnitt 12), welche bei der Dilatation weniger stark belastet werden (z. B. relativ flächig an der Gefäßwand anliegende Bereiche).

## Patentansprüche

1. Stent mit einer Struktur aus einem biokorrodierbaren metallischen Werkstoff, die aus einer Vielzahl miteinander in Verbindung stehender Stegabschnitte besteht, wobei
(i) die Struktur eine Stützstruktur aus einer Anzahl miteinander in Verbindung stehender erster Stegabschnitte aufweist, die ausgelegt sind eine nach einer Expansion des Stents die Gefäßwand abstützende oder die mechanische Integrität des Stents bewahrende Funktion für einen vorgebbaren Zeitraum wahrzunehmen; und
(ii) wenigstens ein zweiter Stegabschnitt vorhanden ist,
(a) der direkt mit einem ausgewählten ersten Stegabschnitt der Stützstruktur elektrisch leitend verbunden ist,
(b) der keine nach der Expansion des Stents die Gefäßwand abstützende oder die mechanische Integrität des Stents bewahrende Funktion für den vorgegebenen Zeitraum wahrnimmt und
(c) dessen Elektrodenpotential E₂ durch eine mechanische Beanspruchung des zweiten Stegabschnittes während oder vor der Expansion des Stents derart herabgesetzt ist, dass es geringer ist als ein Elektrodenpotential E₁ des ausgewählten ersten Stegabschnitts nach der Expansion des Stents.

2. Stent nach Anspruch 1, bei dem der biokorrodierbare metallische Werkstoff eine Legierung eines Elementes ausgewählt aus der Gruppe Magnesium, Eisen und Wolfram ist.

3. Stent nach Anspruch 2, bei dem der biokorrodierbare metallische Werkstoff eine Magnesiumlegierung ist.
